Europäisches Patentamt

(19) European Patent Office

Office européen des brevets

(11) Veröffentlichungsnummer: **0 227 904**
B1

(12) **EUROPÄISCHE PATENTSCHRIFT**

(45) Veröffentlichungstag der Patentschrift:
17.10.90

(51) Int. Cl.⁵: **C07C 209/14, C07C 211/03**

(21) Anmeldenummer: 86114624.9

(22) Anmeldetag: 22.10.86

(54) Verfahren zur Herstellung von Trialkylaminen.

(30) Priorität: 06.11.85 DE 3539266

(43) Veröffentlichungstag der Anmeldung:
08.07.87 Patentblatt 87/28

(45) Bekanntmachung des Hinweises auf die Patenterteilung:
17.10.90 Patentblatt 90/42

(84) Benannte Vertragsstaaten:
BE DE FR GB IT NL SE

(56) Entgegenhaltungen:
US-A- 2 609 394
US-A- 3 128 311
US-A- 4 404 404

(73) Patentinhaber: BASF Aktiengesellschaft,
Carl-Bosch-Strasse 38, D-6700 Ludwigshafen(DE)

(72) Erfinder: Müller, Herbert, Dr., Carostrasse 53,
D-6710 Frankenthal(DE)
Erfinder: Fischer, Roman, Dr., Deidesheimer Strasse 1,
D-6704 Mutterstadt(DE)
Erfinder: Jeschek, Gerhard, Dr., Im Zaunrücken 14,
D-6718 Grünstadt(DE)
Erfinder: Schoenleben, Willibald, Dr.,
Blumenthalstrasse 41, D-6900 Heidelberg(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Herstellung tertiärer Trialkylamine mit bis zu insgesamt 9 Kohlenstoffatomen, durch Umsetzung von Dialkylaminen mit $C_2$-$C_4$-Alkoholen in Gegenwart von Hydrierungs-/Dehydrierungskatalysatoren, die als katalytisch hydrierendes/dehydrierendes Metall im wesentlichen nur Kupfer enthalten.

Die katalytische Alkylierung von Aminen mit Alkoholen ist bekannt. Bei dieser Reaktion werden einerseits dehydratisierende Oxide, z.B. die des Aluminiums, Thoriums, Wolframs und Chroms als Katalysatoren verwendet, andererseits sind Hydrierungs- bzw. Dehydratisierungskatalysatoren etwa auf der Grundlage von Kupfer, Nickel, Kobalt und Chrom empfohlen worden. Es sind Verfahren in flüssiger Phase sowie in der Gasphase bekannt. In einer Arbeit von V.A. Nekrasova und N.I. Shuikin in der Zeitschrift Russian Chemicals Reviews, 34, 843 (1965) sowie in dem Buch "The Acyclic Aliphatic Tertiary Amines", L. Spialter und J.A. Pappalardo, The Macmillan Comp., 1965, ise eine ausführliche Darstellung des Sachgebietes abgehandelt. Besonders schwierig ist die Herstellung von tertiären Aminen durch Alkylierung von sekundären Aminen mit Alkoholen. Als Folge von Transalkylierungs- und Disproportierungsreaktionen treten sekundäre und primäre Amine als Nebenprodukte auf, die den gewünschten Eigenschaften der tertiären Amine abträglich sind und aus den Reaktionsprodukten nur schwer abgetrennt werden können.

Deshalb wurde, um die tertiären Amine mit hohen Ausbeuten herstellen zu können, in der DE-A-1 493 781 ein Verfahren beschrieben, bei dem mindestens stöchiometrische Mengen des sekundären Amins mit dem Alkohol umgesetzt werden. Es soll also das umzusetzende Amin in der Reaktionszone in wenigstens äquimolarer Menge mit dem Alkohol vorliegen. Es wird gezeigt, daß ein Überschuß an Alkohol eine schlechte Selektivität hinsichtlich der gebildeten tertiären Amine zur Folge hat. Zwar scheint man bei der Umsetzung von sekundären Alkoholen mit dieser Maßnahme eine gewisse Verbesserung der Selektivität des Amins zu erreichen, der Umwandlungsgrad des Amins ist jedoch gering. Bei primären Alkoholen werden jedenfalls geringe Umsätze und eine schlechte Selektivität beobachtet, denn als Nebenprodukte entstehen Rückstände, die die Ausbeute vermindern.

Ein verbessertes Verfahren zur Herstellung von tertiären Aminen beschreibt US-A 3 708 539; dabei wird in flüssiger Phase ein Alkohol mit einem sekundären Amin an Katalysatoren auf der Grundlage von Ruthenium, Osmium, Rhenium oder Technetium zur Reaktion gebracht. Nachteil dieses Verfahrens ist der Umstand, daß Umsatz und Ausbeute, bezogen auf die ein gesetzten Alkohole, gemessen am Wert der Katalysatorrohstoffe, für ein technisches Verfahren unzureichend sind.

Besonders tertiäre Amine vom Typ Dimethylfettalkylamin, deren Fettalkyl-Rest im allgemeinen eine C-Zahl größer 8 aufweist, besitzen besonderes technisches Interesse. Diese Produkte werden nach den verschiedensten Technologien, sei es in der Gasphase, sei es in der Flüssigphase, in großem Umfang hergestellt.

Größere Schwierigkeiten bereitet die Synthese der niedermolekularen tertiären Trialkylamine. Prototypen dieser Verbindungsklasse sind z.B. das Dimethylethylamin und Triethylamin. Diese tertiären Amine werden industriell in großem Umfang benützt um als Katalysator die Polymerisation von Polyurethanharzen zu beschleunigen; z.B. in Kernsandbindemitteln. Weitere tertiäre Amine dieser Produktklasse sind z.B. n-Propyldimethylamin, Methylethyln-propylamin, Isopropyldimethylamin oder n-Butyldimethylamin. Alle Versuche diese niedermolekularen tertiären Amine nach der Methode herzustellen, die bei der Synthese der langkettigen Amine zum Erfolg führte, scheiterten bislang. Deshalb werden in der DE-A-2 838 184 und in der EP-B-24 225 vorgeschlagen, die Synthese dieser Verbindungen entweder an Kupfer-Chromit-Katalysatoren oder reinen Kupferkatalysatoren, die durch thermische Zersetzung und Reduktion von basischen Kupfer-Aluminium-Carbonaten entstehen, durch Reaktion in der Gasphase z.B. aus Dimethylamin und den niedermolekularen Alkoholen herzustellen. Gesundheitliche Gründe sprechen gegen die Verwendung von Chromit-Katalysatoren, da diese cancerogen sind.

Obwohl nach beiden obigen Verfahren die Synthese von z.B. Dimethylethylamin in technisch zufriedenstellender Weise auszuführen ist, bleibt die Synthese weiterhin verbesserungswürdig.

Beiden Verfahren haftet der Nachteil an, daß für die Durchführung der Gasphasenreaktion ein verhältnismäßig hoher Energieaufwand für das Verdampfen der Reaktionskomponenten aufgewendet werden muß, eine verhältnismäßig große Menge Wasserstoff und gasförmiges Dimethylamin im Kreis gefördert werden müssen, und das Zielprodukt, beispielsweise Dimethylethylamin, nur durch großen Kondensationsaufwand aus der geförderten Kreisgasmenge gewonnen werden kann. Meist ist eine hintereinander geschaltete mehrstufige Kondensation erforderlich. Da diese Verfahren in der Gasphase bei relativ niedrigen Wasserstoffpartialdrücken an den nicht nur hydrierend sondern auch dehydrierend wirkenden Katalysatoren durchgeführt werden, bildet sich aus dem Alkohol auch der entsprechende Aldehyd, der durch Aldolkondensation zu unerwünschten Nebenprodukten führt, welche nicht nur die Ausbeute mindern, sondern auch als Verunreinigungen im Endprodukt erscheinen. Im Falle der Synthese des Ethyldimethylamins enthält der Rohaustrag z.B. Acetaldehyd. Das in der EP-B-24 225 beschriebene Verfahren sieht deshalb vor, daß das primär entstandene Reaktionsprodukt vor der endgültigen Aufarbeitung noch einer gesonderten Hydrierung zurgeführt wird, in der Acetaldehyd zum Ethanol hydriert wird, um Aufarbeitungsschwierigkeiten zu vermeiden. Die Ausbeuten, mit denen man die relativ hochwertigen Amine erhält, liegen zwar schon mit über 90 % der Theorie sehr hoch, sind aber angesichts des Wertes, den diese Amine darstellen, weiter verbesserungswürdig.

Es ist ein gravierender Nachteil bei der Synthese des Dimethylethylamins in der Gasphase, daß sich aus dem Synthesebaustein Ethanol der Aldehyd und dessen Kondensationsprodukte als Nebenprodukte bilden. Diese müssen in einer besonderen Hydrierstufe hydriert werden, damit sie aus den Umsetzungsprodukten entfernt werden können bzw. als Alkohol wieder in die Reaktion zurückgeführt werden können. Weil diese zusätzliche Hydrierung schwierig ist, schlägt die EP-B 24 225 ersatzweise auch vor, die Aldehyde mit Hilfe von Hydrazinhydrat durch Bildung von Hydrazonen aus dem Reaktionsgemisch zu entfernen.

Mit der Bildung der Aldehyde als Nebenbestandteile steht die Bildung von inerten Substanzen im Reaktionskreisgas der Gasphaseverfahren im Zusammenhang. Die Aldehyde neigen nämlich zum Zerfall durch Decarbonylierung in Olefin, Wasserstoff und Kohlenmonoxid. Aus diesem Grunde entstehen inerte Gase die sich im Kreisgas anreichern und kontinuierlich ausgeschleust werden müssen. Der Verlust an Wasserstoff ist deshalb bei diesen Verfahren verhältnismäßig hoch.

Die Entstehung der Aldehyde in den Gasphasenverfahren ist möglicherweise der Grund dafür, daß die Lebensdauer der Katalysatoren verhältnismäßig gering ist. In der EP-B-24 225 ist auf Seite 3, in Zeile 46 und 47 die maximale Lebensdauer einer Katalysatorcharge mit 3 Monaten beschrieben.

Aus der US-A 4 404 404 ist es ferner bekannt, aliphatische Alkohole oder Aldehyde mit Ammoniak, primären Aminen oder sekundären Aminen zu den höher substituierten Aminen umzusetzen, wobei als Katalysator eine trägerfreie Mischung aus Kupferoxid oder -hydroxid und einem Oxid oder Hydroxid eines Elementes der Gruppe IIA des Periodensystems verwendet wird. Nach der Lehre dieser Patentschrift (vgl. Spalte 3, 1. Absatz) kommen für diese Reaktion Alkohole bzw. Aldehyde mit 8 und mehr C-Atomen in Betracht.

Die Herstellung von Trialkylaminen nach diesem Verfahren, ausgehend von den wesentlich reaktiveren Alkoholen geringerer Kohlenstoffzahl, gelingt wegen diverser Nebenreaktionen aber nur sehr unbefriedigend.

Der Erfindung liegt die Aufgabe zugrunde, ein Verfahren zur Herstellung tertiärer Trialkylamine mit bis zu insgesamt 9 Kohlenstoffatomen, durch Umsetzung von Dialkylamin mit $C_2$-$C_4$-Alkoholen in Gegenwart von Hydrierungs-/Dehydrierungskatalysatoren, die als katalytisch hydrierendes/dehydrierendes Metall im wesentlichen nur Kupfer enthalten, zur Verfügung zu stellen, das die zuvor beschriebenen Nachteile nicht aufweist. Es soll ein Verfahren der genannten Art bereitgestellt werden, das die Herstellung tertiärer Trilalkylamine, insbesondere z.B. Ethyldimethylamin, ohne Ausbeuteminderung bei nur geringem thermischem und mechanischem Energieaufwand erlaubt. Dazu ist es erforderlich, daß auf die sonst allgemein empfohlene Kreisgasfahrweise, sei es in der Gasphasenreaktion entsprechend den Anmeldungen DE-A 2 838 184 und EP-B 24 225 oder in der Flüssigphasenumsetzung gemäß den in der DE-B 2 535 073 und JP-A 222448/1984 beschriebenen Verfahren, verzichtet wird.

Diese Aufgabe wird gelöst mit einem Verfahren der genannten Art, das dadurch gekennzeichnet ist, daß die Reaktionspartner

a) in flüssiger Phase vorliegen,
b) Alkali- und/oder Erdalkalioxide und/oder -hydroxide enthalten,
c) im Molverhältnis Dialkylamin : Alkohol = 1:10 bis 10:1 und
d) in Anwesenheit des bei der Reaktion gebildeten Wassers umgesetzt werden.

Das erfindungsgemäße Verfahren läßt sich in einfachen, unkomplizierten und deshalb billigen Reaktionsapparaten mit praktisch quantitativen Ausbeuten durchführen. Der hierbei erforderliche Energieaufwand kann gering gehalten werden. Die einzusetzenden Katalysatoren sind einfach und billig und weisen eine lange Lebensdauer auf. Beim erfindungsgemäßen Verfahren wird die Bildung unerwünschter Nebenprodukte unterdrückt und man erhält das Wertprodukt mit hoher Selektivität. Das bei der Reaktion entstandene Wasser verbleibt im Reaktionsansatz ohne in unerwarteter Weise die Reaktion hinsichtlich Ausbeute oder Geschwindigkeit zu beeinträchtigten. Vielmehr steigen Ausbeute und Reaktionsgechwindigkeit an.

Im Gegensatz zum Verfahren der EP-B 24 225 werden bei der Alkylierung des Dialkylamins Aldehyde überhaupt nicht oder nur in so untergeordneter Menge gebildet, daß sie aus dem Reaktionsaustrag nicht eigens entfernt werden müssen. Anders als beim bekannten Verfahren wird bei Anwendung eines niedrigen Wasserstoffpartialdrucks praktisch kein Wasserstoff verbraucht. Das gebildete Wasser muß nicht durch aufwendige Kreisgasfahrweise kostenintensiv aus dem Reaktionsgemisch entfernt werden.

Man verwendet beim erfindungsgemäßen Verfahren reine Kupferkatalysatoren entweder im Festbett oder suspendiert und bringt die Reaktionspartner in flüssiger Phase in Gegenwart von ebenfalls in der flüssigen Phase gelöstem oder suspendiertem Alkali- und/oder Erdalkalioxid und/oder -hydroxid zur Umsetzung. Man kann als Alkali- bzw. Erdalkalioxid bzw. -hydroxid Oxide bzw. Hydroxide des Lithiums, Natriums, Kaliums, Magnesiums, Calciums, Strontiums, Bariums, bzw. deren Mischungen, einsetzen.

Wichtig ist auch, daß der Wasserstoffpartialdruck im Reaktionsgemisch im Vergleich zum Gesamtdruck niedrig gehalten wird.

Im Gegensatz zu anderen, früher beschriebenen Verfahren, ist es erfindungsgemäß nicht notwendig, das bei Umsetzung gebildete Wasser aus dem Reaktionsgemisch laufend zu entfernen um eine vollständige Umsetzung des Dialkylamins zu erreichen. Besonders dieses letztere Ergebnis war bei der Umsetzung in flüssiger Phase nicht zu erwarten, da es bekannt ist, daß in anderen Fällen (vgl. z.B. DE-C 2 625 196) die vollständige Umsetzung nur gelingt, wenn Wasser im Maße seiner Bildung aus dem Reaktionsgemisch entfernt wird. Dies steht im Einklang mit dem Reaktionsmechanismus nach welchem die Bildung der tertiären Amine abläuft (hydrolytische Rückspaltung gebildeter Zwischenprodukte).

Nach dem erfindungsgemäßen Verfahren können insbesondere einwertige aliphatische Alkohole mit der Kohlenstoffzahl 2-4 wie Ethanol, n-Propanol, i-Propanol, n-Butanol oder sec.-Butanol mit Dialkylaminen, gegebenenfalls in Gegenwart von Wasserstoff, bei nahezu quantitativer Ausbeute zu den tertiären Trialkylaminen umgesetzt werden. Umalkylierung und Bildung von sekundären oder gar primären Aminen werden nur in nicht zu beachtenden, geringem Umfang beobachtet.

Als Dialkylamine können sekundäre Amine wie Dipropylamin, Ethylmethylamin, Diethylamin, Dimethylamin oder z.B. auch Methylbutylamin eingesetzt werden.

Das Verfahren kann an Festbettkatalysatoren nach der sogenannten Sumpf- oder Rieselfahrweise bzw. auch an suspendierten Katalysatoren ausgeübt werden.

An Festkatalysatoren arbeitet man zweckmäßig folgendermaßen:

Als Reaktor verwendet man z.B. ein senkrecht angeordnetes zylindrisches Gefäß, mit den üblichen Einrichtungen zur Kühlung oder Heizung und der Zufuhrmöglichkeit der Reaktionsteilnehmer. Das Reaktionsgefäß ist mit dem Katalysator, der beliebig geformt sein kann, gefüllt. Häufig verwendet man den Katalysator als Strangpreßling mit einem Durchmesser von 3-6 mm und einer Länge bis zu 5 cm. Es kann aber auch tablettiert in Form von Zylindern mit den Maßen von z.B. 5 mm Durchmesser und 5 mm Höhe oder als Kugeln oder in jeder anderen beliebigen Form eingesetzt werden.

Wendet man die Rieselfahrweise an, so wird das den Katalysator enthaltende Reaktionsgefäß von oben nach unten mit den Reaktionspartnern Dialkylamin und Alkohol beaufschlagt z.B. mit einer Geschwindigkeit von 200-500 mm Reaktionsgemisch pro Liter Katalysatorvolumen und Stunde, und man stellt den gewünschten Reaktionsdruck, der sich aus dem Dampfdruck der Reaktionspartner und Reaktionsprodukte, einem geringen Wasserstoffpartialdruck und gegebenenfalls zusätzlich verwendetem Inertgasdruck zusammensetzt, ein. Auf den Auslaß von Abgas kann weitgehend verzichtet werden, da sich nach dem erfindungsgemäßen Verfahren fremde Inertgase praktisch nicht bilden. Insofern ist das Verfahren in Bezug auf den Wasserstoffverbrauch besonders ökonomisch. Am Ofenende wird das Reaktionsprodukt einer Vorlage, die standgeregelt betrieben wird, kontinuierlich entnommen. Da die Reaktion mit ca. 7 Kcal/mol leicht exotherm ist, kann das Reaktionsgefäß isotherm gehalten werden, indem man einen Teil des Umsetzungsprodukts mittels einer Kreispumpe nach vorhergehender Kühlung auf die Katalysatorschüttung zurückführt. Da man die Reaktionsführung meist so vornimmt, daß entweder das eingesetzte Dialkylamin oder der Alkohol vollständig umgesetzt wird, kann man nunmehr das gewünschte tertiäre Trialkylamin in reiner Form gewinnen und den überschüssig eingesetzten Reaktionspartner nach destillativer Abtrennung vom Reaktionswasser erneut für weitere Umsetzungen verwenden.

Neben dieser eben beschriebenen und bevorzugten Anwendung der Rieselfahrweise läßt sich die Umsetzung auch dergestalt durchführen, daß die Reaktionspartner von unten nach oben in flüssiger Form das Katalysatorbett als sogenannten Sumpf durchströmen. Auch hier kann Reaktionswärme gegebenenfalls durch Rückführung abgeführt werden. Wie bei der Rieselfahrweise ist es auch möglich, durch den im Überschuß eingesetzten Reaktionspartner die Reaktionsführung isotherm ablaufen zu lassen.

In der bevorzugten Ausführungsform des erfindungsgemäßen Verfahrens verwendet man Kupferkatalysatoren, die in der DE-A 2 445 303 beschrieben worden sind. Sie können als amorphe Produkte der thermischen Zersetzung und Reduktion basischer Kupfer-Aluminium-Carbonate angesehen und dadurch erhalten werden, daß man verdünnte oder mäßig konzentrierte, zweckmäßig unter 3-molare Lösungen von Kupfer- und Aluminiumsalzen mit Alkalicarbonat bei pH 8-10 fällt und die erhaltenen Niederschläge vor oder nach entsprechender Formung bei einer Temperatur von 350-600°C zersetzt. Nach üblicher Reduzierung, bevorzugt in Gegenwart des bei der späteren Umsetzung verwendeten Alkohols, erhält man hochaktive, für das vorliegende Verfahren bestens geeignete Katalysatoren. Es können aber auch Kupferkatalysatoren verwendet werden, die man durch Tränken von geeignetem Trägermaterial z.B. Bimsstein, Diatomeenerde, Kieselgel, Thorium- oder Aluminiumoxid, sowie nachfolgende Reduktion erhält.

Bei der nach dem erfindungsgemäßen Verfahren ebenfalls möglichen Suspensionsfahrweise ist der reduzierte Kupferkatalysator in den Reaktionskomponenten Alkohol und Dialkylamin suspendiert. Geeignete Katalysatoren sind z.B. Raney-Kupfer oder die oben beschriebenen Kupferkatalysatoren in gepulverter Form. Bevorzugt ist jedoch ein aktives Kupfermaterial, das man dadurch erhält, daß man Kupferformiat in Gegenwart eines Alkohols und Dialkylamin auf 200-250°C erhitzt. Die Bildungsweise eines solchen Katalysators wird z.B. in der EP-B 70 512 beschrieben.

Die erfindungsgemäße Synthese wird in Gegenwart eines Überschusses an Alkohol oder sekundärem Amin durchgeführt. Der Überschuß soll wenigstens die zweifache molare Menge betragen. Nach oben hin ist der Überschuß nur entsprechend wirtschaftlichen Gesichtspunkten zu begrenzen. Da eine Ausbeutesteigerung bei einem Molverhältnis Amin : Alkohol bzw. Alkohol : Amin wie 1:5 kaum noch zu beobachten ist, wird man deswegen die Reaktion im allgemeinen mit einem Molverhältnis Amin : Alkohol wie 1:5 bis 5:1, vorzugsweise 1:4 bis 4:1 vornehmen.

Eine sehr wichtige erfindungsgemäße Maßnahme beim vorliegenden Verfahren besteht darin, den flüssigen Reaktionspartnern ein Erdalkali- und/oder Alkalioxid bzw. -hydroxid zuzugeben. Durch diese Maßnahme gelingt es Umalkylierungsreaktionen und Disproportionierungen völlig zu unterdrücken. Dabei war es überraschend, daß diese Wirkung nicht eintritt, wenn die Alkali- bzw. Erdalkaliverbindungen bereits den Katalysatoren, z.B. bei der Herstellung von Formkörpern zugesetzt werden. Offensichtlich ist es unerläßlich, daß die Erdalkali- oder Alkali-Verbindungen den Reaktionspartnern am Katalysator in kontinuierlicher Weise zur Verfügung gestellt werden. Diese Aussage trifft natürlich nur für die kontinuierliche Ausführung der erfindungsgemäßen Verfahrens zu, sei es am Festbettkatalysator oder z.B. an suspendierten Katalysatoren in einer Reaktionskaskade.

Die Reaktion wird in einem Temperaturbereich von 160 bis 250°C durchgeführt. Der bevorzugte Bereich beträgt 170 bis 210°C.

Die Reaktionsgeschwindigkeit ist sehr hoch, es lassen sich ohne weiteres 100 bis 300 Gew.-Teile tertiäres Trialkylamin pro Liter Katalysatorvolumen in der Stunde erzeugen.

Bei der Durchführung des erfindungsgemäßen Verfahren stellt man in überraschender Weise fest, daß die Katalysatoren über außerordentlich lange Zeiträume ihre Aktivität unvermindert erhalten. In der Festbettkatalyse z.B. kann an dem bevorzugten Kupferkatalysator gemäß der DE-A 2 445 303 mehr als 1 Jahr lang gearbeitet werden. Das gleiche gilt für das aktive Kupfermetall, das man aus Kupferformiat als Katalysator gewinnt.

Die folgenden Beispiele dienen zur weiteren Erläuterung des erfindungsgemäßen Verfahrens ohne die Erfindung zu beschränken. Die genannten Teile sind Gewichtsteile; sie verhalten sich zu Volumenteilen wie kg zu Liter.

Beispiel 1

Für die kontinuierliche Herstellung des Ethyldimethylamins wurde verflüssigtes Dimethylamin technischer Qualität und mit 1,2 % Toluol vergälltes Ethanolazeotrop (95,6 % Ethanol, 4,4 % Wasser) verwendet.

Die Aminierung wurde in einem senkrecht stehenden Reaktionsrohr mit 1.000 Volumenteilen Reaktionsinhalt durchgeführt. Das Verhältnis des Durchmessers zur Länge des Reaktionsrohres betrug 1:40. Mittels eines organischen Wärmeträgers, der in einem Heizmantel umgepumpt wurde, ließ sich das Reaktionsrohr thermostatisieren. In das Reaktionsrohr waren 700 Volumenteile eines Katalysators gefüllt, der die Form von Zylindern hatte, die 3 mm Höhe und 3 mm Durchmesser maßen. Der Katalysator war nach der Vorschrift des Beispiels 1 der DE-A2 445 303 hergestellt worden. Der Katalysator wurde bei 180-200°C mit Wasserstoff reduziert, wobei gleichzeitig stündlich 300 ml Ethanol über den Katalysator gerieselt wurden. Die Reduktion wurde zunächst mit einem Wasser/Stickstoff-Gemisch (50 bar), später mit reinem Wasserstoff durchgeführt, bis im abfließenden Ethanolazeotrop kein Reduktionswasser mehr nachweisbar war. Dem Ofen wurden dann bei 200°C stündlich 300 Teile Ethanol, das 0,15 % Natriumhydroxid enthielt und 100 Teile Dimethylamin, bei 50 bar Gesamtdruck (Wasserstoffpartialdruck ca. 10 bar) von oben nach unten zugefahren. Am Kopf der Vorlage wurden stündlich ca. 0,1 bis 0,2 Volumenteile Wasserstoff als Abgas gefahren. Das die Vorlage verlassende Reaktionsprodukt hatte nach gaschromatographischer Analyse und Destillationsanalyse folgende Zusammensetzung:

Trimethylamin:1 Gew.%
Monomethylamin:1 Gew.%
Dimethylamin:1 Gew.%
Dimethylethylamin:50 Gew.%
Diethylmethylamin:1 Gew.%
Ethanol:32 Gew.%
Toluol1,5 Gew.%

Durch Destillation läßt sich aus diesem Rohgemisch Dimethylethylamin mit über 99,5 % Reinheit gewinnen.

Ein ähnliches Ergebnis wird erzielt, wenn dem Ethanol statt Natriumhydroxid Kaliumhydroxid oder Lithiumhydroxid zugesetzt wurde.

Einen ähnlichen, wie oben beschrieben zusammengesetzten Reaktionsaustrag erhielt man, wenn man anstelle des oben beschriebenen Fällkatalysators einen Kupfertränkkatalysator verwendete, der 20 % Kupferoxid auf Kieselgel enthielt und mit etwa der Hälfte des wie oben beschrieben zusammengesetzten Zulaufs beaufschlagt wurde.

Beispiel 2

Ein Rührbehälter mit einem Reaktionsvolumen von 2.000 Teilen wurde mit 920 Teilen Ethanol, 180 Teilen Dimethylamin, 5,5 Teilen Calciumhydroxid und 55 Teilen metallischem Kupferkatalysator beschickt.

Der Kupferkatalysator war zuvor in einer getrennten Reaktion aus Kupferformiat bei 200°C in Gegenwart von mit Dimethylamin gesättigtem Laurylalkohol hergestellt worden. Bei 20°C wurden auf den Rührbehälter 10 bar Wasserstoff aufgepreßt und anschließend auf 210°C aufgeheizt. Der Gesamtdruck des Reaktionssystems stieg dabei auf 55-60 bar an. Man rührte das System 6 Stunden lang bei Reaktionstemperatur, leiß dann abkühlen, den Katalysator sich absetzen und drückte die überstehende klare Lösung ab. Sie hatte etwa folgende Zusammensetzung:

Monomethylamin:0,2 Gew.%
Trimethylamin:0,2 Gew.%
Dimethylamin:0,3 Gew.%
Dimethylethylamin:28 Gew.%
Diethylmethylamin:0,2 Gew.%
Unbekannte:0,7 Gew.%
Ethanol:60 Gew.%
Toluol1,6 Gew.%
Wasser10 Gew.%
Höhersieder:0,2 Gew.%

Aus dem Reakrtionsaustrag ließ sich Dimethylethylamin in einer Reinheit von über 99.5 % durch fraktionierte Destillation gewinnen.

Beispiel 3

Es wurde in der im Beispiel 2 beschriebenen Reaktionsapparatur mit 52 Teilen des im Beispiel 1 beschriebenen pulverisierten Katalysators und 2,5 Teilen 50 %iger Natronlauge 925 Teile n-Butanol und 103 Teile Di methylamin bei 50 bar Gesamtdruck (Partialdruck des Reaktionsgemisches + Wasserstoffpartialdruck) bei 220°C miteinander zur Umsetzung gebracht. Man rührte 10 Stunden lang bei Reaktionstemperatur, ließ dann abkühlen und isolierte wie in Beispiel 2 beschrieben das Reaktionsprodukt, das sich wasserfrei gerechnet wie folgt zusammensetzte:

Monomethylamin:0,1 Gew.%
Trimethylamin:0,9 Gew.%
Dimethylamin:0,1 Gew.%
Dimethylbutylamin:15,9 Gew.%
Dibutylmethylamin:0,3 Gew.%
n-Butanol-1:82,7 Gew.%

Beispiel 4

Man arbeitete wie in Beispiel 3 beschrieben und setzte in Gegenwart von 62 Teilen des im Beispiel 1 beschriebenen pulverisierten Katalysators und 2,5 Teilen 50 %iger Natronlauge 744 Teile Diethylamin mit 95 Teilen Ethanol bei 50 bar Gesamtdruck (Partialdruck der Reaktionsmischung + Wasserstoffpartialdruck) und 210°C um. Man rührte 10 Stunden lang bei Reaktionstemperatur, ließ dann abkühlen und isolierte dann wie beschrieben das Reaktionsprodukt, das wasserfrei gerechnet folgende gaschromatographische Zusammensetzung besaß:

Unbekannte:0,3 Fl.%
Monoethylamin:3,1 Fl.%
Diethylamin:47,9 Fl.%
Triethylamin:47,6 Fl.%
Ethanol: 1,1 Fl.%

## Patentansprüche

1. Verfahren zur Herstellung von Trialkylaminen mit bis zu insgesamt 9 Kohlenstoffatomen, durch Umsetzung von Dialkylaminen mit $C_2$–$C_4$-Alkoholen in Gegenwart von Hydrierungs-/Dehydrierungskatalysatoren, die als katalytisch hydrierendes/dehydrierendes Metall im wesentlichen nur Kupfer enthalten, dadurch gekennzeichnet, daß die Reaktionspartner
   a) in flüssiger Phase vorliegen,
   b) Alkali- und/oder Erdalkalioxide und/oder -hydroxide enthalten,
   c) im Molverhältnis Dialkylamin : Alkohol = 1:10 bis 10:1 und
   d) in Anwesenheit des bei der Reaktion gebildeten Wassers umgesetzt werden.
2. Verfahren nach Anspruch 1, dadurch gekennzeichnet, daß die Reaktionspartner im Molverhältnis Dialkylamin : Alkohol = 1:4 bis 4:1 umgesetzt werden.
3. Verfahren nach Ansprüchen 1 oder 2, dadurch gekennzeichnet, daß die Reaktion bei Temperaturen im Bereich von 160 bis 250°C durchgeführt wird.
4. Verfahren nach einem der Ansprüche 1 bis 3, dadurch gekennzeichnet, daß man in Gegenwart von Wasserstoff arbeitet.
5. Verfahren nach einem der Ansprüche 1 bis 4, dadurch gekennzeichnet, daß die Reaktion bei einem Gesamtdruck von 40 bis 250 bar und einem Wasserstoff-Partialdruck von 2 bis 30 bar durchgeführt wird.
6. Verfahren nach einem der Ansprüche 1 bis 5, dadurch gekennzeichnet, daß man einen Kupfer-Festbettkatalysator verwendet.
7. Verfahren nach Anspruch 6, dadurch gekennzeichnet, daß man einen Katalysator einsetzt, der durch thermische Zersetzung und Reduktion eines basischen Kupfer-Aluminiumcarbonats erhältlich ist, wobei dieses Kupfer-Aluminiumcarbonat durch Fällen einer Kupfer- und Aluminiumsalze enthaltenden Lösung bei pH 8 bis 10 gebildet wurde.
8. Verfahren nach einem der Ansprüche 1 bis 5, dadurch gekennzeichnet, daß man in Gegenwart eines suspendierten Kupferkatalysatoren arbeitet.
9. Verfahren nach Anspruch 8, dadurch gekennzeichnet, daß man einen Kupferkatalysator verwendet, der durch Erhitzen von Kupferformiat in Gegenwart von Alkohol und Dialkylamin auf Temperaturen oberhalb 170°C erhältlich ist.

## Claims

1. A process for preparing a trialkylamine having a total of up to 9 carbon atoms by reacting a dialkylamine with a $C_2$-$C_4$-alcohol in the presence of a hydrogenation/dehydrogenation catalyst which contains essentially only copper as the hydrogenating/dehydrogenating catalytic metal, wherein the reactants
   a) are present in the liquid phase,
   b) contain alkali metal and/or alkaline earth metal oxides and/or hydroxides and
   c) are reacted in a molar ratio of dialkylamine to alcohol of from 1:10 to 10:1 and
   d) in the presence of the water formed during the reaction.
2. A process as claimed in claim 1, wherein the molar ratio of dialkylamine to alcohol in the reaction is from 1:4 to 4:1.
3. A process as claimed in claim 1 or 2, wherein the reaction is carried out at from 160 to 250°C.
4. A process as claimed in any of claims 1 to 3, which is carried out in the presence of hydrogen.
5. A process as claimed in any of claims 1 to 4, wherein the reaction is carried out under a total pressure of from 40 to 250 bar and under a hydrogen partial pressure of from 2 to 30 bar.
6. A process as claimed in any of claims 1 to 5, wherein a fixed bed copper catalyst is used.
7. A process as claimed in claim 6, wherein the catalyst used is obtainable by thermal decomposition and reduction of a basic copper aluminum carbonate, the latter being formed by precipitating a solution containing copper and aluminum salts at pH 8-10.
8. A process as claimed in any of claims 1 to 5, which is carried out in the presence of a suspended copper catalyst.
9. A process as claimed in claim 8, wherein the copper catalyst used is obtainable by heating copper formate in the presence of an alcohol and a dialkylamine at above 170°C.

## Revendications

1. Procédé de préparation de trialkylamines ayant jusqu'à 9 atomes de carbone au total, par réaction de dialkylamines avec des alcools en $C_2$–$C_4$ en pré-

sence de catalyseurs d'hydrogénation/déshydrogénation qui contiennent essentiellement seulement du cuivre comme métal catalytique d'hydrogénation/déshydrogénation, caractérisé en ce que les partenaires réactionnels

a) sont en phase liquide,

b) contiennent des oxydes et/ou hydroxydes alcalins et/ou alcalino-terreux,

c) sont mis en réaction dans un rapport molaire dialkylamine : alcool de 110 à 10:1 et

d) en présence de l'eau formée dans la réaction.

2. Procédé selon la revendication 1, caractérisé en ce que les partenaires réactionnels sont mis en réaction dans un rapport molaire dialkylamine : alcool de 1:4 à 4:1.

3. Procédé selon les revendications 1 ou 2, caractérisé en ce que la réaction est menée à des températures comprises entre 160 et 250°C.

4. Procédé selon l'une quelconque des revendications 1 à 3, caractérisé en ce qu'on travaille en présence d'hydrogène.

5. Procédé selon l'une quelconque des revendications 1 à 4, caractérisé en ce que la réaction est menée à une pression totale de 40 à 250 bar et à une pression partielle d'hydrogène de 2 à 30 bar.

6. Procédé selon l'une quelconque des revendications 1 à 5, caractérisé en ce qu'on utilise un catalyseur au cuivre en lit fixe.

7. Procédé selon la revendication 6, caractérisé en ce qu'on utilise un catalyseur qui a été obtenu par décomposition thermique et réduction d'un carbonate de cuivre et d'aluminium basique, ce carbonate de cuivre et d'aluminium ayant été formé par précipitation d'une solution contenant des sels de cuivre et d'aluminium à un pH de 8 à 10.

8. Procédé selon l'une quelconque des revendications 1 à 5, caractérisé en ce qu'on travaille en présence d'un catalyseur au cuivre en suspension.

9. Procédé selon la revendication 8, caractérisé en ce qu'on utilise un catalyseur au cuivre qui a été obtenu par chauffage de formiate de cuivre en présence d'alcool et de dialkylamine à des températures supérieures à 170°C.